# FASCICULE DE BREVET EUROPEEN

(11) **EP 1 207 367 B1**
(45) Date de publication et mention de la délivrance du brevet: **22.07.2009**
(21) Numéro de dépôt: 01440392.7
(22) Date de dépôt: 19.11.2001
(51) Int. Cl.: G01B 11/24

(54) **Installation et procédé automatiques de prise de mensurations et d'acquisition de formes**
Automatische Installation und Verfahren für Gestaltmessungen und -ermittlungen
Automatic installation and method for measurement and detection of shapes

(30) Priorité: 20.11.2000 FR 0014963
(43) Date de publication de la demande: 22.05.2002
(73) Titulaire: Telmat Industrie (Société Anonyme), 68360 Soultz (FR)
(72) Inventeur: Dudkiewicz, Gilbert, 68270 Ruelisheim (FR); Keiter, Pascal, 68740 Hirtzfelden (FR); Guerlain, Philippe, 68630 Bennwihr (FR); Noel, Franck, 68320 Urschenheim (FR)
(74) Mandataire: Nuss, Pierre

(56) Documents cités:
- EP-A- 0 683 379

## Description

La présente invention concerne le domaine de la prise de mesures et de l'acquisition de représentations de sujets, en particulier de sujets humains, et a pour objet une installation automatique de prise de mensurations et d'acquisition de formes de sujets, ainsi qu'un procédé permettant de réaliser automatiquement ces opérations.

Différents systèmes d'acquisition de forme tridimensionnelle de sujets et de prises de mensurations, notamment en vue de leur classification, existent déjà, tels que par exemple ceux divulgués par les documents GB-A-2159943 et EP-A-0 524 119.

Toutefois, ces appareils et dispositifs connus présentent tous un ou plusieurs des inconvénients suivants : structure complexe, impossibilité de réaliser des reconstitutions tridimensionnelles précises et réalistes du sujet, nécessité d'un étalonnage fastidieux avant chaque acquisition ou après chaque modification de positionnement des moyens de prise de vues, protection insuffisante contre la luminosité parasite et fiabilité réduite conséquente des mesures, impossibilité d'exploitation simultanée et corrélative de plusieurs vues du même sujet compte tenu des durées d'acquisition et des mouvements du sujet dans ces intervalles, acquisition d'informations partielles du fait de la couleur et de la nature des vêtements portés par le sujet, nécessité de bouger ou de faire bouger le sujet pour l'acquisition de plusieurs vues et donc non reproductibilité de conditions de prise de vue identiques, nécessité d'acquérir, de traiter et de stocker une masse importante de données pour la fourniture de données précises et la production d'une reproduction tridimensionnelle virtuelle du sujet, procédé non entièrement automatique, notamment au niveau de l'étalonnage.

La présente invention a notamment pour but de surmonter au moins certains des inconvénients et certaines des limitations précités.

Elle a notamment pour but de proposer un système et un procédé permettant des prises de mensurations et des acquisitions de formes tridimensionnelles de sujet de manière automatique, rapide, fiable et précise, sans nécessiter de dispositifs de prise de vues trop complexes ou des moyens de traitement et de stockage de très grande capacité, en s'affranchissant des interférences lumineuses ambiantes, de la couleur et de la nature des vêtements portés par le sujet et des procédures d'étalonnage fastidieuses et répétitives avec manipulation d'objets étalons.

A cet effet, la présente invention selon la revendication I a pour objet une installation automatique d'acquisition de formes tridimensionnelles et de prise de mensurations de sujets, notamment sous la forme d'une cabine destinée à des sujets humains, comprenant au moins une unité de projection de franges, en particulier de franges lumineuses, et au moins une unité de prise de vues rapide, ainsi qu'une unité de commande de l'installation et de traitement des vues acquises, caractérisée en ce que lesdites unités de projection et de prise de vues sont regroupées dans une ou plusieurs tête(s) d'acquisition et en ce que ladite installation comprend également, d'une part, plusieurs marqueurs ou points de repérage fixes et plusieurs surfaces de projection de référence entourant la zone d'acquisition et de mesure recevant le sujet à saisir et situés dans le champ de vision de l'unité ou de chacune des unités de prise de vues et, d'autre part, un moyen de génération d'un mur de lumière temporaire à l'opposé de ladite au moins une tête d'acquisition ou de chacune des têtes d'acquisitions par rapport à la zone d'acquisition.

Selon la revendication II, la présente invention a également pour objet un procédé automatique d'acquisition quasi-instantanée de formes tridimensionnelles et de prise de mensurations de sujets, en particulier humains, notamment au moyen de l'installation précitée, caractérisé en ce qu'il consiste essentiellement, à placer ou faire placer un sujet au niveau d'une zone d'acquisition, cette zone comportant des marqueurs et des surfaces de projection de référence, situés dans les champs de vision de têtes d'acquisition de vues dudit sujet et constituant des repères d'étalonnage permanents, à réaliser l'acquisition d'une vue dite vue de face comprenant, d'une part, la prise d'une première image en vue de face du sujet au moyen d'une première unité de prise de vues en synchronisme avec une illumination créant un mur de lumière derrière le sujet par l'intermédiaire d'un premier moyen de génération de mur de lumière, pour l'acquisition d'un premier contour de silhouette en ombre chinoise et, d'autre part, la prise d'une seconde image de devant du sujet au moyen de ladite première unité de prise de vues en synchronisme avec la projection d'un réseau de franges horizontales au moyen d'une première unité de projection sur la face avant du sujet, puis à réaliser l'acquisition d'une vue dite vue de derrière ou de dos comprenant, d'une part, la prise d'une première image en vue de dos du sujet au moyen d'une seconde unité de prise de vues en synchronisme avec une illumination créant un mur de lumière devant le sujet par l'intermédiaire d'un second moyen de génération de mur de lumière, pour l'acquisition d'un second contour de silhouette en ombre chinoise, et, d'autre part, la prise d'une seconde image de dos du sujet au moyen de ladite seconde unité de prise de vues en synchronisme avec la projection d'un réseau de franges horizontales au moyen d'une seconde unité de projection sur la face arrière du sujet et, enfin, à traiter, au moyen d'une unité de commande et de traitement adapté, les images et les vues acquises par corrélation et exploitation des différentes informations fournies par elles en vue de la constitution d'une représentation tridimensionnelle du sujet de l'extraction de mensurations et/ou de la classification du sujet dans une ou des catégories prédéfinies.

L'invention sera mieux comprise, grâce à la description ci-après, qui se rapporte à un mode de réalisation préféré, donné à titre d'exemple non limitatif, et expliqué avec référence aux dessins schématiques annexés, dans lesquels :
les figures 1A et 1B des vues schématiques par transparence du dessus et de côté d'une installation selon un premier mode de réalisation préféré de l'invention ;
la figure 2 est une représentation de l'image vue à travers l'optique de l'un des dispositifs de prise de vues faisant partie de l'installation représenté sur la figure 1 ;
la figure 3 est une représentation similaire à celle de la figure 2 avec projection du réseau de franges selon l'invention et correction de certaines déformations de l'image ;
les figures 4A et 4B sont des vues schématiques partielles en élévation latérale d'une installation selon l'invention comprenant deux variantes de réalisation d'un moyen de génération de mur de lumière ;
la figure 5 est une représentation du contour de silhouette d'un sujet humain féminin obtenue en vue de face grâce à l'installation selon l'invention ;
la figure 6 représente schématiquement les différentes phases de prises de vues possibles conformément au procédé selon l'invention ;
les figures 7A et 7C représentent respectivement l'image acquise par projection de franges après détourage, l'image obtenue après extraction des extrema du signal le long de lignes verticales parallèles et l'image obtenue après identification des franges ou fragments de franges réel(le)s et suppression des artefacts ;
la figure 8 est une représentation théorique du réseau de franges projeté ;
les figures 9A et 9C sont des représentations partielles du signal selon la ligne C-C' de la figure 8 respectivement au moment de la projection du réseau de franges, au moment de l'acquisition optique de l'image fournie par le réseau de franges projetés et après échantillonnage ;
les figures 10A à 10D sont des représentations schématiques illustrant différentes étapes de l'opération d'identification de fragments de franges par l'intermédiaire de la relation d'adjacence mise en oeuvre dans le cadre du procédé selon l'invention ;
les figures 11A et 11B représentent, pour un plan donné au niveau de la poitrine d'un sujet humain, les coupes de face et de dos, avant et après jonction ;
la figure 12 est une représentation d'un sujet sous. forme d'empilement de coupes ;
les figures 13 et 14 représentent schématiquement, respectivement en vue de côté et en vue de dessus, des variantes de réalisation de l'installation représentée sur les figures 1, et,
la figure 15 est un ordinogramme représentant le déroulement d'une séance d'acquisition mettant en oeuvre l'installation selon l'invention sous forme de cabine destinée à des sujets humains.

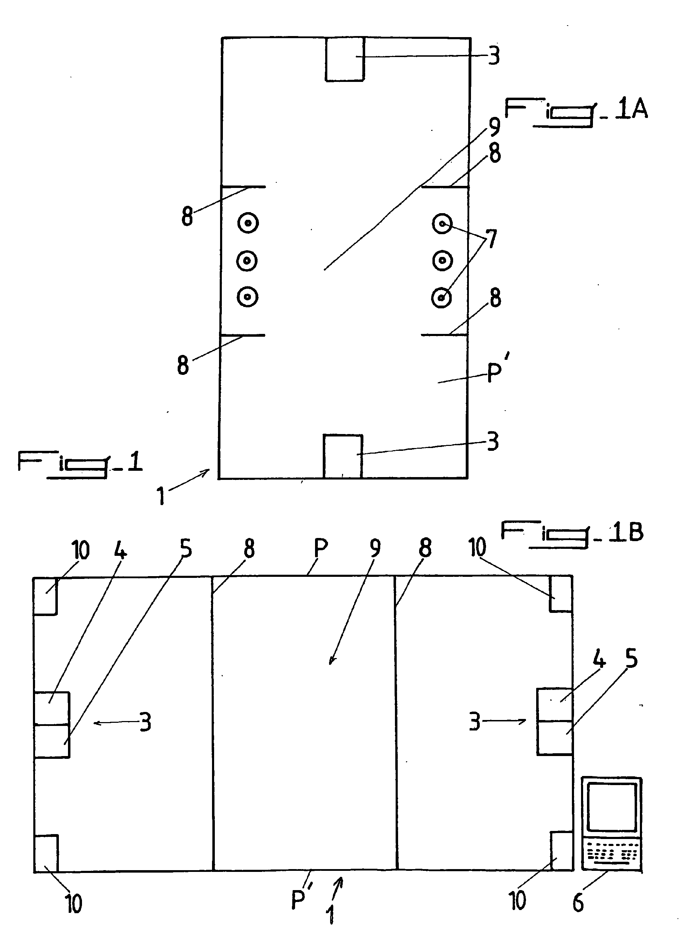

Comme le montrent notamment les figures 1, 2, 3 et 6 des dessins annexés, l'installation automatique 1, notamment sous la forme d'une cabine destinée à des sujets 2 humains, comprend au moins une unité 4 de projection de franges, en particulier lumineuses, et au moins une unité 5 de prise de vues rapide, ainsi qu'une unité 6 de commande de l'installation et de traitement des vues acquises, intégrée ou séparée de ladite installation 1.

Conformément à l'invention, les unités 4, 5 de projection et de prise de vues sont regroupées dans une ou plusieurs tête(s) d'acquisition 3 et ladite installation 1 comprend également, d'une part, plusieurs marqueurs ou points de repérage fixes 7 et plusieurs surfaces de projection 8 de référence, par exemple planes, entourant la zone 9 d'acquisition et de mesure recevant le sujet 2 à saisir et situés dans le champ de vision de l'unité ou de chacune des unités 5 de prise de vues et, d'autre part, un moyen 10 de génération d'un mur de lumière temporaire à l'opposé de ladite au moins une tête d'acquisition 3 ou de chacune des têtes d'acquisitions 3 par rapport à la zone d'acquisition 9.

Les murs de lumière permettent de masquer la structure de la cabine 1 à l'image et de fournir une luminosité suffisante pour faire ressortir des contours nets malgré la luminosité ambiante.

Selon une première caractéristique de l'invention, ressortant notamment des figures 1, 2 et 3, la zone d'acquisition 9 consiste en un volume sensiblement parallélépipédique, les marqueurs 7 étant disposés à des emplacements connus de l'unité de commande et de traitement 6 selon plusieurs alignements latéraux dans la direction de prise de vue et dans deux plans parallèles espacés P, P' délimitant les faces supérieure et inférieure dudit volume 9 et les surfaces de projection de référence 8, non déformantes et préférentiellement planes, consistant en des réglettes verticales reliant les deux plans P, P' précités au niveau des quatre arêtes verticales de la zone d'acquisition 9, les informations fournies par les marqueurs 7 et les surfaces de projection 8 sur les différentes vues étant exploitées par l'unité 6 à chaque acquisition en vue de l'étalonnage de l'installation 1.

Les marqueurs 7 pourront par exemple consister en des disques ou des pastilles circulaires sombres présentant un motif en forme de cible ou de mire ou une pastille centrale plus claire et leurs alignements pourront par exemple s'étendre entre les réglettes 8, en dehors de la partie centrale de la zone 9 recevant le sujet 2, de manière à ne pas être cacher sur les images.

En outre, des arrangements ou un nombre de marqueurs 7 différents dans les deux plans P et P' permettront à l'unité 6 de reconnaître sans difficulté l'orientation de l'image acquise.

Les marqueurs 7, préférentiellement disposés de manière symétrique par rapport à un plan médian vertical de la cabine, seront détectés automatiquement par l'installation 1 à chaque démarrage et au cours de chaque séance d'acquisition et permettront de déterminer l'ensemble des paramètres extrinsèques des unités de prise de vues 5 à prendre en compte par l'unité de commande et de traitement 6 pour le contrôle de l'installation 1 et l'exploitation des vues.

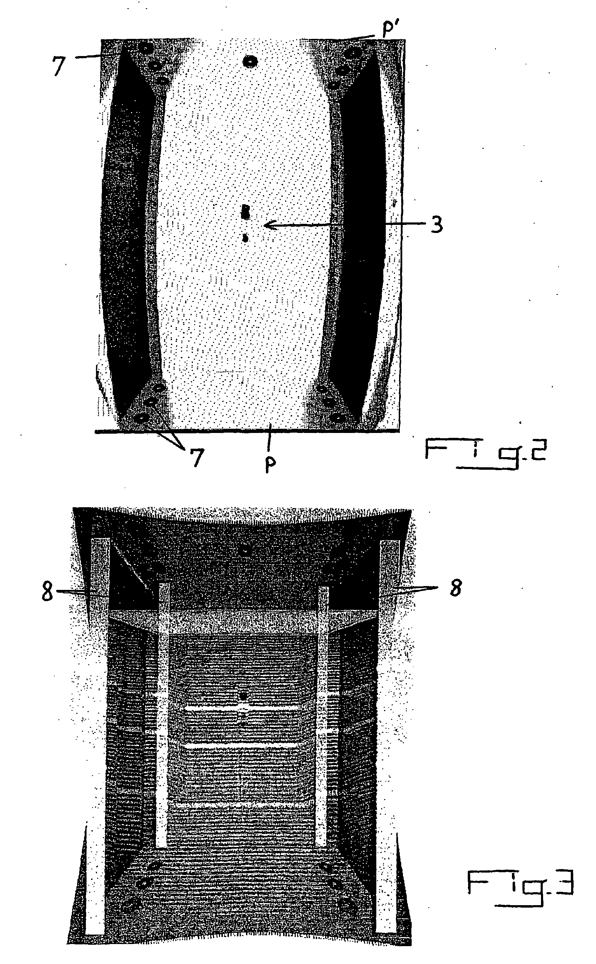

Les réglettes 8, préférentiellement sous forme de plaques allongées vues selon des dispositions similaires par les unités 5, permettent du fait de leur répartition spatiale aux coins de la zone 9, de localiser et d'identifier chaque plan de frange (voir figure 3) et peuvent éventuellement servir de montants plans pour soutenir la structure de la cabine 1 et définir des passages pour entrer et sortir de cette dernière.

Lesdits marqueurs 7 et réglettes ou montants 8 permettent un étalonnage et une acquisition précis du fait de leurs situations en périphérie de la zone d'acquisition 9, et autorisent la réalisation d'une étape d'étalonnage et de calibration à chaque acquisition de vue, leur prise en compte par l'unité n'étant pas gênée par le sujet 2.

En effet, la prise de vue effectuée par l'installation 1 ne se réduit pas à une prise de photo au sens commun du terme puisqu'il faut identifier la transformation entrant en jeu lors du passage du monde réel au monde image et corriger les déformations introduites dans l'image acquise par le système optique.

Une fois l'image corrigée, il est possible d'en extraire le contour nécessaire à une partie de la prise de mesure ainsi que les franges projetées sur le corps qui serviront à la reconstruction tridimensionnelle de la surface.

Les franges sont ensuite couplées aux paramètres de la transformation identifiée lors de la prise de vue afin de reconstruire l'image tridimensionnelle de la surface photographiée.

Le bon fonctionnement de ce processus réside dans la bonne modélisation mathématique des transformations successives subies par l'image de la personne lors de la prise de photo. Il faut donc s'attacher à déterminer comment se fait le passage du monde réel au monde image puis, du monde image au monde réel.

Les informations du monde réel tridimensionnel sont acquises par au moins une unité 5 du type caméra vidéo numérique du type CCD (Charge Coupled Device - Dispositif à Couplage de Charge), associé à un objectif susceptible de couvrir la totalité du volume d'acquisition 9 sans déplacement.

Grâce aux moyens d'étalonnage précités, l'unité 6 est en mesure de corriger la distorsion radiale de l'image ainsi que la rotation de la caméra par rapport aux repères de la cabine. A la suite de l'étalonnage en usine et de l'étalonnage sur site, les images de travail obtenues sont directement exploitables pour effectuer des calculs de longueur ou de positionnement à l'image.

Les paramètres du modèle de caméra étant fixés, il est possible d'obtenir les coordonnées images (u ; v) de tout point (x ; y ; z) de la scène. Inversement, il est possible de calculer les coordonnées (x ; y ; z) d'un point de la scène sur la seule base de ses coordonnées images (u ; v) et d'une indication sur son positionnement en z.

Les unités de prise de vues 5, préférentiellement au nombre d'au moins deux, seront disposées selon des configurations autorisant la prise en compte de la totalité ou de la quasi-totalité de la surface du corps du sujet 2 sans déplacement de ce dernier, ni desdites unités 5.

Selon un mode de réalisation préférentiel de l'invention et comme le montrent les figures 1A et 1B des dessins annexés, l'installation 1 comporte deux têtes d'acquisition 3 situées en opposition de part et d'autre de la zone d'acquisition 9, et chacune desdites têtes 3 englobe dans son champ de vision l'ensemble des marqueurs 7 et des surfaces de projection 8. De plus, à chacune desdites têtes 3 est associé un ou des moyen(s) 10 de génération d'un mur de lumière correspondant, l'arrangement des marqueurs 7 et/ou des surfaces de projection 8 permettant de déterminer les extrémités supérieures, inférieures et latérales de la zone d'acquisition 9, ainsi que l'orientation de cette dernière.

Lesdites unités 3 seront avantageusement disposées à mi-hauteur environ, de manière à ce que la seconde unité 3 soit couverte lors d'une prise de vue par la première unité 3 et vice versa et qu'elles soient centrées par rapport au volume 9.

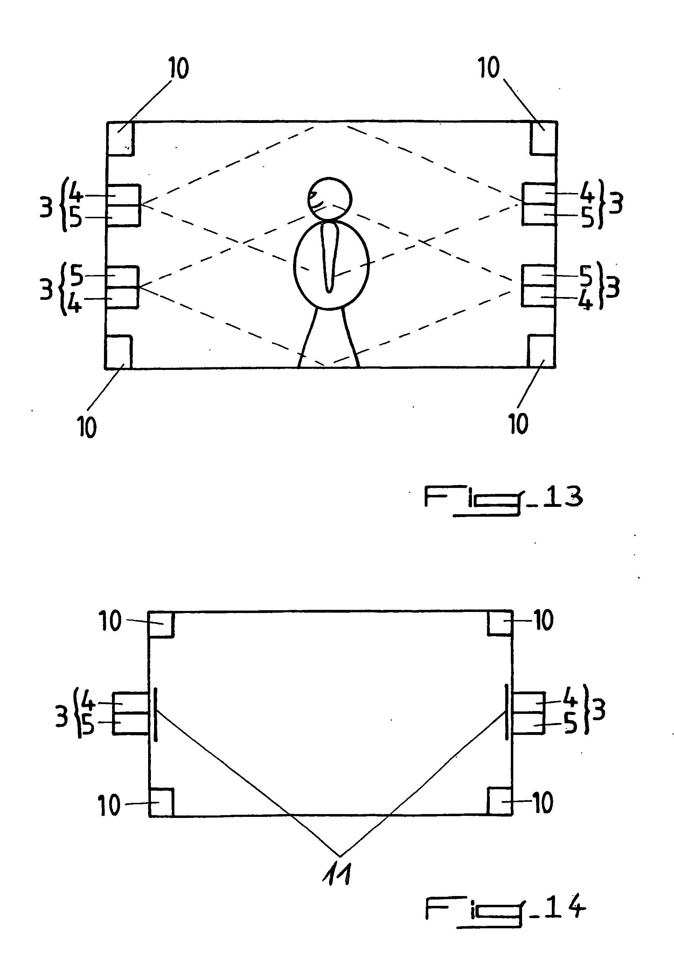

Conformément à une variante de réalisation de l'invention, représentée à la figure 13 des dessins annexés, l'installation 1 peut comporter quatre têtes d'acquisition 3 regroupées en deux paires de têtes situées en opposition de part et d'autre de la zone d'acquisition 9. Une tête, située dans une position inférieure, de chacune des paires de têtes précitées englobe dans son champ de projection et de vision une partie inférieure du volume d'acquisition 9 et l'autre tête, située sans une position supérieure, de chacune desdites paires de têtes englobe une partie supérieure dudit volume 9, complémentaire de et partiellement chevauchante avec la partie inférieure précitée, lesdites têtes inférieures, respectueusement supérieures, englobant, en outre, les marqueurs 7 inférieurs, respectivement supérieurs, et des portions inférieures, respectivement supérieures, des surfaces de projection 8 de référence.

Une telle disposition permet d'acquérir les vues avec une définition plus importante et donc de disposer de vues et de représentations plus précises, le traitement des informations étant similaire à celui mis en oeuvre en relation avec deux têtes d'acquisition 3 simples, à l'exception d'une élimination des informations redondantes au niveau des portions chevauchantes et d'un assemblage des parties inférieures et supérieures des vues de face et de dos.

On remarquera sur la figure 13, que les dispositions relatives des unités 4 et 5 des têtes inférieures et supérieures de chaque paire sont inversées.

Selon deux autres variantes de réalisation de l'invention, non représentées aux dessins annexés, l'installation 1 peut comporter soit au moins trois têtes d'acquisition 3, ou au moins trois unités de prises de vues 5, disposées en Y dans la cabine formant l'installation 1, soit au moins quatre têtes d'acquisition 3, ou au moins quatre unités de prises de vues 5, disposées en X dans la cabine formant l'installation 1.

Les trois ou quatre têtes d'acquisition 3 pourront également consister en des paires de têtes comme décrites précédemment pour la disposition en I.

Ces deux variantes permettent d'obtenir des angles de vues latéraux (et non frontaux) et donc de recueillir des informations supplémentaires concernant les faces latérales du sujet 2.

De plus, les unités 4 et/ou 5 pourront être montées dans la cabine 1 (Fig 1 et 13) ou être montées à l'extérieur de cette dernière en étant séparées de l'intérieur de la cabine par une partie de cloison transparente 11 (figure 14).

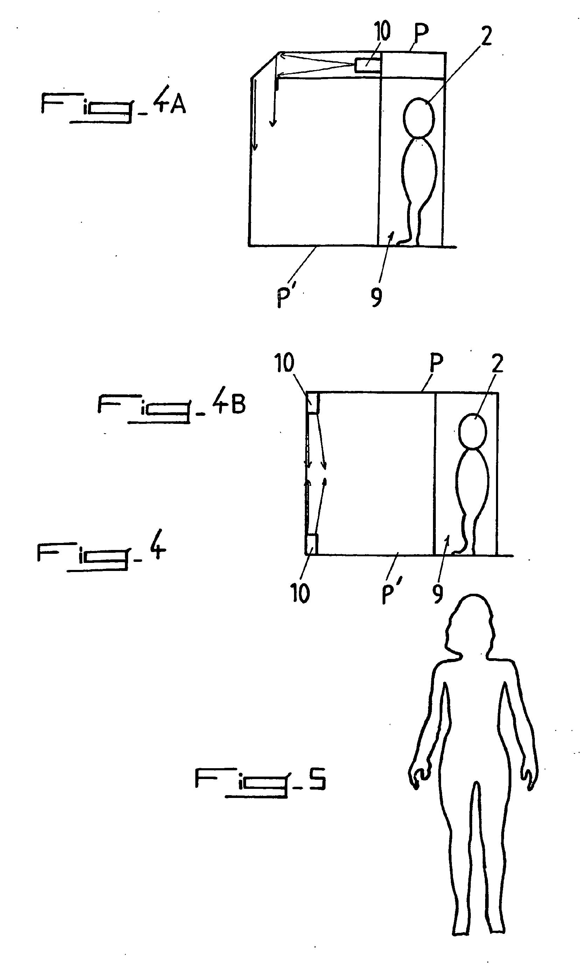

Selon une caractéristique de l'invention, représentée aux figures 1B et 4 des dessins annexés, les moyens 10 de génération de mur de lumière consistent en des flashs électroniques dont le déclenchement est synchronisé avec celui de l'unité ou des unités de prise de vues 5 qui lui est (sont) opposée(s) par rapport à la zone d'acquisition 9, cette ou ces unité(s) 5 consistant chacune en une caméra vidéo numérique, par exemple du type CCD.

Cette disposition permet, en centrant la période d'acquisition des caméras 5 par rapport à l'intervalle d'éclairage des flashs 10, d'annuler l'influence de la lumière ambiante pour la prise des images de contours, de limiter la consommation énergique et de ne pas perturber l'acquisition des images obtenues lors de la projection de franges.

La figure 4A montre une réalisation de chaque moyen 10 sous la forme d'un flash logé dans la partie supérieure de la cabine 1 et dont le flux lumineux est dévié par une surface inclinée pour être rasant en fond de cabine et la figure 4B montre une réalisation, similaire à celle de la figure 1, dans laquelle chaque moyen 10 est formé de deux flashs antagonistes disposés à deux extrémités opposées d'un fond de la cabine 1, déclenchés simultanément et dont les flux émis sont dirigés en direction de l'autre flash.

De même, les franges alternées noires et blanches sont produites par les unités 4 au moyen de flashs électroniques associés à des objectifs de projection sans déformation géométrique, par exemple du type asphérique, et synchronisés en déclenchement avec les unités de prise de vues 5 des têtes d'acquisition 3 correspondantes, les franges noires présentant une largeur supérieure à celles des franges blanches ou lumineuses, le rapport des largeurs franges noires / franges blanches étant compris entre 1,1 et 1,5, préférentiellement d'environ 1,3.

Cette dernière disposition permet d'obtenir un bon contraste entre les raies blanches et noires sans saturer les caméras 5.

Afin de pouvoir garantir une identification et une localisation fiable des différentes franges, quelles que soient les déformations introduites par les optiques et par les inégalités de la configuration de la surface du sujet 2 à acquérir tridimensionnellement, chacun des réseaux de franges horizontales projetées comporte au moins deux franges blanches FR1, FR2, FR3 mutuellement espacées, présentant des largeurs nettement supérieures à celles des autres franges, préférentiellement deux ou trois fois plus larges, et servant de franges de référence pour le repérage et l'identification des autres franges.

Préférentiellement, comme le montrent les figures 3, 7 et 8, chacun des réseaux de franges comporte trois franges de référence FR1, FR2, FR3 disposées de manière asymétrique dans le motif du réseau concerné et/ou avec des espacements mutuels non identiques, une desdites franges de référence FR1 étant localisée pour une projection à environ un tiers de la hauteur du volume formé par la zone d'acquisition 9 et au moins une autre FR2 pour une projection à environ deux tiers de la hauteur du volume formé par ladite zone 9.

Dans le cas d'un sujet 2 humain, une desdites franges de référence FR1 sera alors localisée pour une projection au niveau des jambes du sujet 2 placé dans la zone d'acquisition 9 et au moins une autre FR2 pour une projection entre les poignets et les épaules dudit sujet 2.

Les unités 4, 5 et 6, ainsi que les éléments constitutifs de la structure de la cabine (ossature, embase, panneaux blancs constituant les fonds et les plans P et P',...), sont connus de l'homme du métier et ne nécessitent pas une description plus détaillée, leurs caractéristiques particulières ayant été indiquées précédemment.

L'installation 1 comportera, en outre, des moyens de détection de la présence d'un sujet 2 et de détermination de sa position (éventuellement prises de vue nécessaires par les têtes d'acquisition 3 et exploitation à ces fins par l'unité 6), associés à des moyens de restitution de messages sonores destinés à guider et à informer le sujet, permettant de rendre le fonctionnement de l'installation entièrement automatique et autonome.

La vérification préliminaire du positionnement global est un processus qui qualifie la position de la personne dans la cabine et qui diagnostique ainsi si l'acquisition est potentiellement bonne pour réaliser une extraction des contours et des surfaces. Ce processus diagnostique également les mauvaises attitudes des personnes, ce qui permet éventuellement de diffuser un message sonore adéquat pour la correction de l'attitude de la personne.

Il existe également une expertise postérieure à l'acquisition, qui détermine si les surfaces et les contours acquis sont de qualité suffisante pour lancer une extraction de mesures.

Ladite installation pourra, le cas échéant, également être pourvue d'une interface de communication permettant au sujet 2 de dialoguer avec l'unité 6, par exemple pour déterminer la destination des mensurations ou la forme de leur édition (fiche, carte magnétique, télétransmission, ...).

L'invention a également pour objet un procédé automatique d'acquisition quasi-instantanée de formes tridimensionnelles et de prise de mensurations de sujets, en particulier humains, notamment au moyen de l'installation 1 précitée comportant deux têtes d'acquisition 3 opposées, caractérisé en ce qu'il consiste essentiellement, à placer un sujet 2 au niveau d'une zone d'acquisition 9 située dans une cabine 1 ou analogue, ce sujet 2 étant placé dans une posture et/ou une position donnée(s) et cette zone 9 comportant des marqueurs 7 et des surfaces de projection 8 de référence, situés dans les champs de vision de têtes d'acquisition 3 de vues dudit sujet 2 et constituant des repères d'étalonnage permanents, à réaliser l'acquisition d'une vue de face comprenant, d'une part, la prise d'une première image en vue dite vue de face du sujet 2 au moyen d'une première unité de prise de vues 5 en synchronisme avec une illumination créant un mur de lumière derrière le sujet 2 par l'intermédiaire d'un premier moyen 10 de génération de mur de lumière, pour l'acquisition d'un premier contour de silhouette en ombre chinoise et, d'autre part, la prise d'une seconde image de devant du sujet 2 au moyen de ladite première unité 5 de prise de vues en synchronisme avec la projection d'un réseau de franges horizontales au moyen d'une première unité de projection 4 sur la face avant du sujet 2, puis à réaliser l'acquisition d'une vue dite vue de derrière ou de dos prise dans une direction opposée à la direction de prise de vue de la vue de face précitée, ladite acquisition comprenant, d'une part, la prise d'une première image en vue de dos du sujet 2 au moyen d'une seconde unité de prise de vues 5 en synchronisme avec une illumination créant un mur de lumière devant le sujet 2 par l'intermédiaire d'un second moyen 10 de génération de mur de lumière, pour l'acquisition d'un second contour de silhouette en ombre chinoise, et, d'autre part, la prise d'une seconde image de dos du sujet 2 au moyen de ladite seconde unité de prise de vues 5 en synchronisme avec la projection d'un réseau de franges horizontales au moyen d'une seconde unité de projection 4 sur la face arrière du sujet 2 et, enfin, à traiter, au moyen d'une unité 6 de commande et de traitement adapté, les images et les vues acquises par corrélation et exploitations des différentes informations fournies par elles en vue de la constitution d'une représentation tridimensionnelle du sujet 2, de l'extraction de mensurations et/ou de la classification du sujet 2 dans une ou des catégories prédéfinies.

Dans le cas d'un sujet humain, la position la plus favorable pourra être indiquée au sol par des repères et la posture idéale pour l'acquisition est la station debout, avec les bras légèrement écartés du corps et les jambes écartées.

La succession d'étapes d'acquisition indiquée ci-dessus peut être modifiée en réalisant, par exemple, successivement les deux images en ombres chinoises, puis les deux images à franges.

L'extraction des contours en ombre chinoise pourra par exemple être effectuée par une méthode de suivi de contour basée sur les gradients d'intensité lumineuse sur l'image acquise (connue de l'homme du métier).

En outre, les images acquises suite aux projections des réseaux de franges sont, pour faciliter leur traitement, élaguées par élimination des fragments de franges situés en dehors des contours.

Préférentiellement, le procédé consiste également à réaliser, au cours de chaque acquisition de vue, une étape d'étalonnage automatique sur la base des informations fournies par les marqueurs 7 et les surfaces de projection 8 de référence sur les différentes images recueillies et exploitées par l'unité 6.

De manière avantageuse, les réseaux de franges projetés comprennent chacun des franges de référence FR1, FR2, FR3 lumineuses réparties de manière asymétrique dans le motif du réseau concerné et présentant une largeur nettement supérieure à celles des autres franges.

En outre, les prises d'images sont réalisées de manière séquentielle dans un intervalle de temps d'acquisition total suffisamment faible pour éviter toute prise en compte d'un mouvement éventuel du sujet 2.

L'opération de traitement précité consiste notamment à détourer les images de surface acquises par projection de franges au moyen des images correspondantes acquises en ombre chinoise, puis à mettre en évidence et à localiser les franges sur les images par filtrage spatial, en déterminant, analysant et en localisant, éventuellement par extrapolation, les extrema supérieurs et inférieurs du signal recueilli le long d'une pluralité de lignes verticales, puis à identifier les différentes franges, le cas échéant après recomposition à partir de fragments de franges, et notamment les franges ou fragments de franges de référence FR1, FR2, FR3, à numéroter ces franges ou fragments de référence et enfin à propager la numérotation à l'ensemble des franges contenu dans les représentations détourées du sujet 2.

L'identification des fragments de franges consiste à établir des relations d'adjacence verticale entre fragments consécutifs dans la direction verticale des vues et à qualifier ces relations, avec identification ou non de franges ou de fragments de franges réel(le)s, en fonction de la longueur ou du taux de recouvrement entre deux fragments adjacents, de la distance moyenne entre deux fragments adjacents et des couleurs respectives de ces derniers.

En outre, la propagation de la numérotation des franges, après identification et numérotation des franges de référence FR1, FR2, FR3, consiste à définir des chemins de recherche partant du haut de chaque représentation du sujet 2 sur les images de surfaces sous forme d'images à franges détourées et s'étendant vers le bas et/ou les côtés jusqu'aux extrémités des différentes ramifications de la représentation du sujet 2.

Enfin, la constitution de la représentation tridimensionnelle consiste, après identification et numérotation sur les images de surface détourées acquises par projection de franges en vue de face et en vue arrière, à générer des représentations, sous forme d'empilements de coupes, de différentes zones distinctes et complémentaires du sujet 2, zones déterminées au moyen de points caractéristiques localisés sur les contours obtenus à partir des vues en ombre chinoise, à assembler entre elles, pour chacune des zones distinctes précitées, les deux demi-coques correspondantes représentant en vue de devant et vue de derrière, sous forme d'empilements de coupes, une zone donnée du sujet 2 et, enfin, à reconstituer une représentation volumique tridimensionnelle totale par assemblage des empilements de coupes représentant les différentes zones distinctes du sujet 2.

Pour vérifier l'exactitude de la restitution tridimensionnelle et des mensurations calculées, il peut, en outre, être prévu de réaliser, le cas échéant après rotation de 90° du sujet 2 autour d'un axe vertical, une prise de vue de profil par l'intermédiaire d'une unité de prise de vue 3 déclenché en synchronisme avec un moyen 10 de génération de mur de lumière opposé correspondant, afin d'acquérir le contour de la silhouette du sujet 2 en vue de profil.

On décrira ci-après plus précisément, à titre d'exemple et en relation avec les figures 3, 7, 8, 9, 10, 11A, 11B et 12 et avec l'installation 1 représentée sur les figures 1A et 1B, les opérations d'extractions des franges et de reconstruction tridimensionnelle d'un sujet 2.

En procédant à quelques modifications tenant compte des angles de prises de vue et du nombre d'unités 5, ce procédé pourra également être mis en oeuvre en relation avec des têtes d'acquisition 3 disposées en X, en Y ou en I avec deux paires de têtes, telles que décrites dans les variantes précitées.

Afin d'obtenir la reconstruction tridimensionnelle de la personne à mesurer, il est nécessaire de procéder à la reconstruction tridimensionnelle de chacune des franges projetées.

Cette reconstruction suppose que les franges soient localisées dans l'image puis identifiées de façon unique par rapport au modèle du motif projeté. Ces franges sont ensuite mises en correspondance avec leurs caractéristiques mathématiques extraites lors de la phase d'étalonnage.

Afin de faciliter la localisation des franges, on effectue un premier filtrage, en ne cherchant les franges qu'à l'intérieur des contours extraits, de manière à ne reconstruire que des éléments présents sur le corps de la personne.

Bien qu'un premier niveau de filtrage soit effectué, la localisation puis l'identification des franges doivent être réalisés.

Trois problèmes peuvent se présenter :
- une frange peut être absente car mal ou non identifiée.
- un élément de la scène observée, un élastique de vêtement par exemple, peut être mal interprété et confondu avec un frange.
- plusieurs franges peuvent se rejoindre notamment sur les reliefs prononcés du corps tel que la poitrine, le cou ou les côtés.

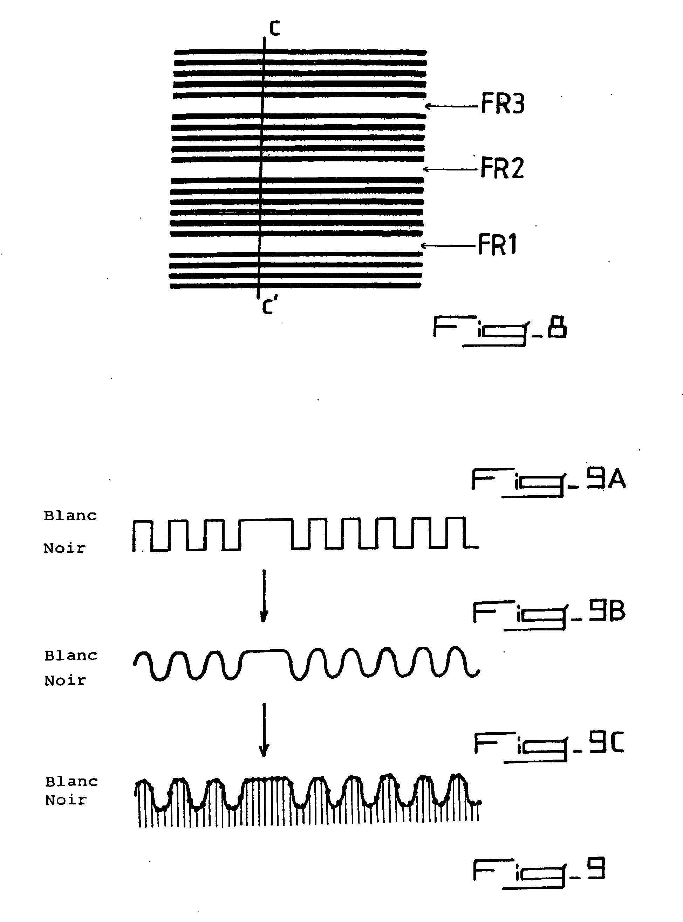

La figure 8 montre un motif de réseau de franges tel que théoriquement projeté et la figure 9A le signal relevé sur une partie de la ligne C-C' de ce motif projeté.

La figure 9B représente le signal (suivant C-C' en partie) tel que recueilli au niveau des caméras 5 et détérioré par les systèmes optiques de projection des franges et d'acquisition des images, après projection du réseau de la figure 9A.

Le signal de la figure 9B est échantillonné par les caméras 5, ou pas de un pixel, tel que représenté sur la figure 9C;

Pour ensuite localiser les franges, il y a lieu de localiser tous les extrema présents à l'image. Etant donné que seule la reconstruction de la surface du sujet est recherchée, on localise uniquement les franges présentes à l'intérieur du contour extrait.

Pour ce faire, il faut balayer l'intérieur du contour colonne par colonne pour y localiser le signal puis recomposer les franges en associant les informations issues du traitement des colonnes.

Les extrema du signal sont localisés au pixel près dans le profil d'intensité du fait de la discrétisation du signal par la caméra. Afin d'obtenir une reconstruction tridimensionnelle des points de franges détectés la plus précise possible, il est impératif de localiser très précisément lesdits extrema.

Pour ce faire, on détermine les extrema théoriques à partir des informations échantillonnées recueillis.

Ayant détecté un extremum dans le signal échantillonné, on suppose que lui, ainsi que ses proches voisins, décrivent une courbe continue d'ordre trois approximant le signal réel (approximation cubique entre quatre points).

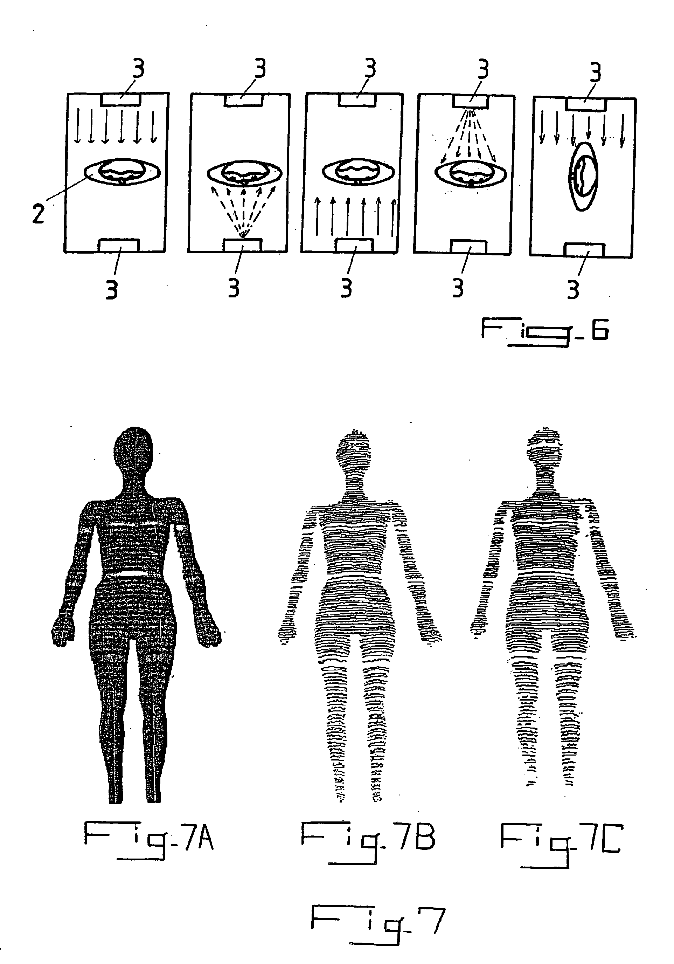

Après détermination de tous les extrema locaux dans toutes les colonnes ou lignes découpant verticalement l'image à franges détourée (minima = franges noires potentielles ; maxima = franges blanches potentielles), comme le montre la figure 7B des dessins annexés, il y a lieu de recomposer les franges auxquelles appartiennent les extrema déterminés.

Pour recomposer les franges, il faut essayer d'associer les extrema de chaque colonne aux extrema des colonnes immédiatement voisines.

Les extrema de deux colonnes immédiatement voisines ne peuvent être associés que s'ils sont de même nature (ils sont tous deux minimum ou maximum). De plus, étant donné qu'une frange apparaît à l'image comme étant une suite de points contigus, l'écart entre leurs ordonnées doit être de un pixel au maximum (étant donné que l'on étudie l'image colonne par colonne, l'écart des abscisses est de toute façon de un pixel).

Cette recomposition ne permet pas toujours d'obtenir des franges continues, on parle alors de fragments de franges. En pratique, les franges sont fragmentées lorsqu'elles apparaissent sur plusieurs parties du corps (par exemple, une frange apparaissant sur les bras et le buste est fragmentée en trois parties). La fragmentation peut être plus importante du fait d'accidents de relief ou de texture.

L'étape suivante consiste à identifier les franges, notamment en éliminant les franges correspondants à des artefacts et non pas à des franges réelles, puis à les numéroter.

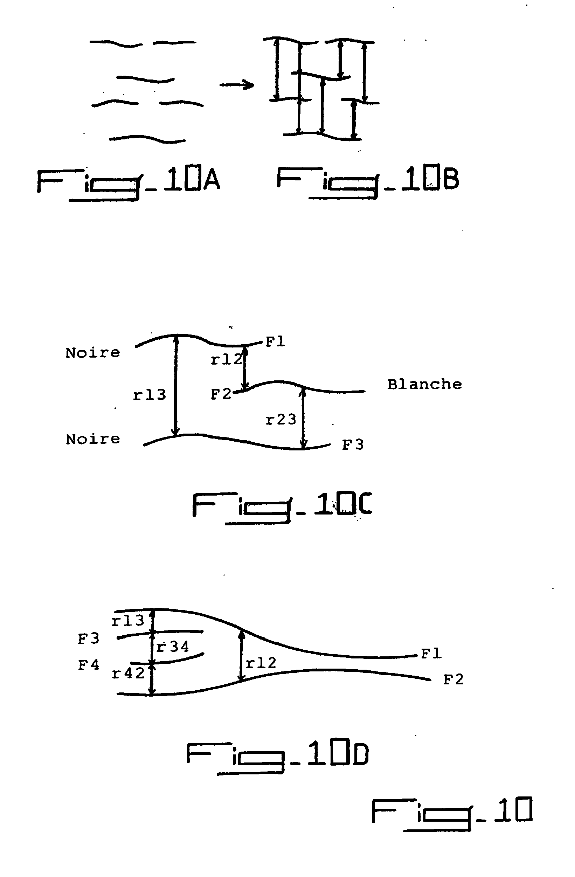

A cet effet, le procédé selon l'invention met en oeuvre, comme déjà indiqué précédemment des relations d'adjacences verticales entre les fragments de franges (voir figures 10A et 10B)

La zone de recouvrement d'un fragment par un fragment adjacent correspond aux points dont les abscisses sont communes à des points du fragment adjacent.

Des zones de recouvrements aux différents fragments adjacents, on peut extraire un taux de recouvrement pour chacun des fragments adjacents selon la formule : Taux de Recouvrement = longueur de recouvrement / longueur du fragment.

Deux fragments adjacents n'auront pas nécessairement, entre eux, les mêmes taux de recouvrement.

La distance moyenne d'un fragment à un de ses fragments adjacents correspond à la moyenne des distances entre les fragments dans la zone de recouvrement.

La couleur des fragments adjacents contribue à déterminer le nombre de fragments éventuellement manquant entre le fragment étudié et ses fragments adjacents.

D'après le schéma de la figure 10C, le fragment F3 est associé aux fragments F1 et F2 par les relations d'adjacence r13 et r23. Etant donné que F1 et F2 sont de couleurs différentes, il est certain que l'écart de numérotation entre F1 et F2 est impair. De même, l'écart de numérotation entre F1 et F3 est pair car les fragments sont de couleurs identiques.

Connaissant la distance moyenne entre deux fragments adjacents, ainsi que l'écart statistique local entre deux fragments, il est possible de faire une estimation de l'écart de numérotation entre ces deux fragments. Pour ce faire, nous calculons le rapport : R = distance moyenne / écart statistique local.

L'écart de numérotation le plus probable correspond alors à l'entier le plus proche de R dont la parité est celle déterminée d'après les couleurs.

A titre d'exemple, supposons que nous ayons obtenu le rapport R = 1,7 entre deux fragments adjacents. Si ces deux fragments sont de couleurs identiques, l'écart de numérotation entre ces deux fragments correspondra au nombre pair le plus proche de R, à savoir 2. Si, par contre, les fragments sont de couleurs différentes, l'écart de numérotation correspondra au nombre impair le plus proche de R, c'est à dire 1.

Les relations d'adjacence entre les fragments sont ensuite qualifiées afin de déterminer quels écarts de numérotations sont les plus fiables. Ces qualifications dépendent de l'importance de l'arrondi dont est issu l'écart, du taux de recouvrement du fragment à son fragment adjacent et de la longueur même du fragment.

Si le fragment adjacent est de relativement petite taille, l'écart de numérotation que l'on en extrait est moins fiable que s'il émanait d'un fragment d'une longueur plus conséquente. Plus le fragment est petit, plus il risque d'être le représentant d'une perturbation locale comme le montre l'exemple de la figure 10D qui correspond à un pli fessier.

Cette méthode permet, sur l'exemple représenté sur la figure 10D, de confirmer le fragment F2 comme successeur du fragment F1, les fragments F3 et F4 étant fort probablement accidentels.

A ce niveau du traitement, on dispose d'un ordonnancement relatif des fragments qui permet à présent d'établir le numéro de chaque fragment de proche en proche. Les fragments de même numéro appartiendront à la même frange.

Pour établir ensuite la numérotation, il est indispensable de disposer d'un ou plusieurs fragments identifiés qui serviront de références.

La première étape de la numérotation consiste à créer, en fonction du contour, des chemins privilégiés de recherche des fragments des franges de référence qui soient adaptés à la morphologie humaine. La caractéristique de ces chemins est de passer sur des zones de l'image correspondantes à des parties réputées peu perturbées du corps. On évite ainsi d'être dérangé par des zones telles que la poitrine et les plis fessiers. La figure 7A montre un exemple de chemins de recherche.

Sur chacun des chemins, on sélectionne des fragments qui pourraient être des fragments de référence. Un fragment de référence est un fragment blanc encadré par deux fragments noirs qui sont distants d'une valeur proche de quatre fois l'écart entre fragment moyen.

Parmi tous les fragments retenus, certains sont sélectionnés alors qu'ils ne sont pas des fragments de référence tandis que d'autres, qui sont des fragments de référence, ne sont pas sélectionnés pour diverses raisons de dégradation du signal.

Il faut ensuite filtrer la sélection obtenue afin d'éliminer les fragments qui ne correspondent pas statistiquement à la description du motif tout en palliant éventuellement à l'absence dans la sélection de l'un ou l'autre fragment de référence. La comparaison des sélections de fragments de référence obtenus sur les divers chemins permet de sélectionner des configurations qui garantissent la cohérence de l'ensemble de la numérotation.

Les fragments de référence FR1, FR2 et FR3 étant identifiés, il faut leur attribuer leurs numéros en fonction des données connues du motif.

Il reste à présent à propager la numérotation à l'ensemble des fragments depuis chacun des fragments de référence en suivant les relations d'adjacence.

Lors de la numérotation d'un fragment par un fragment adjacent, un indice de qualité sur la numérotation est également propagé. L'indice de qualité est calculé en fonction de l'indice de qualité du fragment adjacent d'où provient la numérotation ainsi qu'en fonction de la qualité de la relation d'adjacence. L'indice de qualité est maximum pour un fragment de référence et se dégrade au fur et à mesure de la propagation de la numérotation.

La numérotation attribuée à un fragment peut être différente selon les fragments adjacents car leurs numérotations ne sont pas issues des mêmes fragments de référence et n'ont pas forcément suivies les mêmes relations d'adjacence. Le fragment conservera alors le numéro qui lui a été attribué avec la meilleure qualité.

Cette opération de numérotation achevée, il est possible de procéder à la reconstitution tridimensionnelle du sujet 2.

La reconstruction du sujet 2, par exemple du corps d'un sujet humain consiste à construire un volume fermé à l'aide des images de surfaces de face et de dos. Pour ce faire, on adopte le principe d'un corps constitué d'un empilement de coupes tridimensionnelles dont les caractéristiques de hauteur et d'inclinaison sont paramétrables.

Toutefois, les points des franges supports des surfaces de face et de dos ne sont pas des points dont la coordonnée Y est constante du fait du relief du corps et de l'angle de projection des franges.

Pour disposer des coupes nécessaires, il faut donc les générer à partir des images de surfaces de face et de dos recueillies.

Pour fermer les coupes précitées, on isole des zones particulières divisant le corps du sujet humain. Pour ce faite, on détecte un minimum de points caractéristiques tels que la base du cou, l'entrejambe et les dessous de bras. Une fois détectés, ces points caractéristiques servent à identifier le buste, les bras, les jambes et la tête dans ces contours.

Les points des contours étant exprimés en coordonnées image, on ne dispose pas de reconstruction tridimensionnelle les concernant, les franges n'y étant pas visibles. Toutefois, ayant conservé les coordonnées image des points des surfaces, il est très facile de connaître la zone de provenance de chaque point des surfaces à l'aide des contours.

Pour reconstruire le corps à l'aide d'un empilement de coupes, il faut, en premier lieu, générer les coupes propres à chaque zone du corps précitée en traitant séparément les coupes dans la surface de face et dans la surface de dos. Une coupe dans une surface est facilement obtenue en parcourant toutes les facettes du maillage et en conservant les facettes intersectées par le plan de coupe.

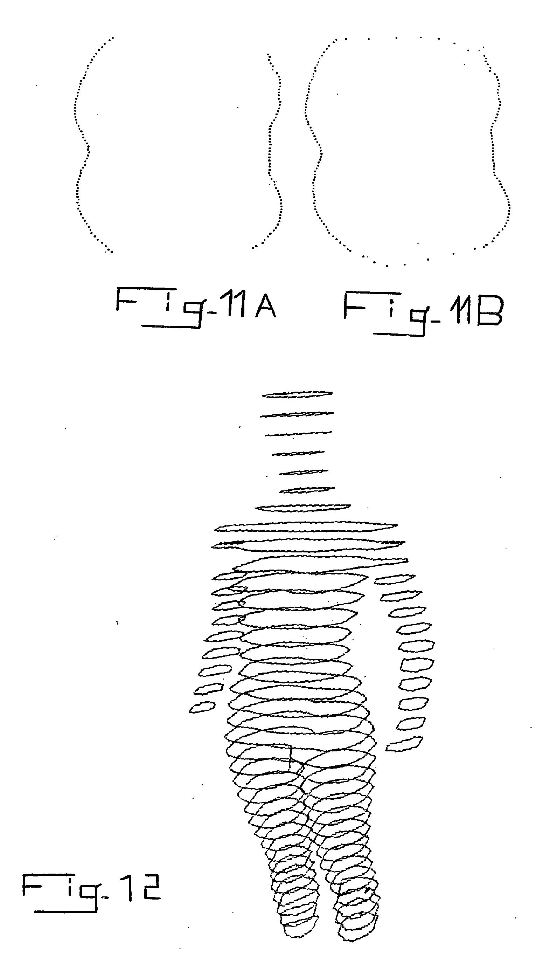

Une fois les coupes de face et de dos extraites (voir figure 11A), il faut les fermer de la façon la plus naturelle possible. En effet, lors de l'acquisition par deux têtes 3 disposées en I, une grande surface située sur les flancs de la personne n'a pas été acquise par les deux têtes 3 car non visible par ces dernières. Afin de ne pas fermer le corps d'une façon trop brutale, ce qui fausserait les mesures, il importe de joindre les coupes de face et de dos à l'aide d'une courbe respectant la forme du corps initial (connaissance a priori de la forme du sujet à l'endroit considéré).

A cet effet, on extrait des points du contour une information de largeur de pixel, qui est utilisée pour fermer les coupes correspondantes auxdits points concernés par l'intermédiaire d'une interpolation cubique (figure 11B).

Une fois les coupes fermées, il faut empiler séparément les coupes propres à chaque zone du corps. Ces assemblages peuvent ensuite être joints après avoir généré les coupes de jonction entre les zones (jonction bras gauche et buste par exemple).

La reconstruction du corps finalement obtenue (figure 12) peut être exportée sous des formats tels que ceux connus sous les désignations DXF (pour la Conception Assistée par Ordinateur - CAO), STL (pour le prototypage rapide) ou AAOP (pour la CAO médicale). Ces formats permettent l'exploitaiton de la reconstruction en CAO ou pour du prototypage rapide de prothèses par exemple. Il est facile d'imaginer que les données acquises soient également transmises à un fabricant de vêtements sur-mesure via Internet.

Le corps reconstruit peut avant tout servir à la prise de mesures. Cette prise de mesures est considérablement simplifiée par rapport aux systèmes existants déformant un mannequin générique. En effet, il s'agit dans le cadre de l'invention de mesurer des circonférences et des longueurs sur ce corps en se basant sur des points précis tels que le saillant des fesses, les mamelons, les dessous de bras, etc.

Ces points sont déterminés en collaboration entre les contours et le corps reconstruit sous forme de représentation tridimensionnelle. Cette corrélation est possible car les coordonnées image des points des franges ont été conservées. On peut ainsi mettre en relation des points du contour avec des points des surfaces acquises et vice-versa. Les contours servent à pré-positionner certains points non visibles sur le corps reconstruit ou plus facilement identifiable sur la silhouette comme l'entrejambe, les dessous de bras ou la base du cou par exemple. Le corps reconstruit, quant à lui, sert à détecter des points saillants ou des ruptures de pente dans les zones délimitées grâce aux points caractéristiques des contours. Etant donné que les points conservent leur niveau de gris d'origine tout au long du traitement, le corps reconstruit permet également de détecter des taches correspondant par exemple au nombril ou à un mamelon.

Pour la majeure partie des mesures de circonférence, il suffit de générer une coupe à un endroit déterminé du corps reconstruit puis d'en mesurer la longueur de l'enveloppe convexe. C'est le cas par exemple du tour de poitrine qui correspond à la longueur de l'enveloppe convexe de la coupe horizontale passant par les mamelons. L'enveloppe convexe des coupes peut être établie selon la démarche dite de Graham appliquée dans le plan de coupe.

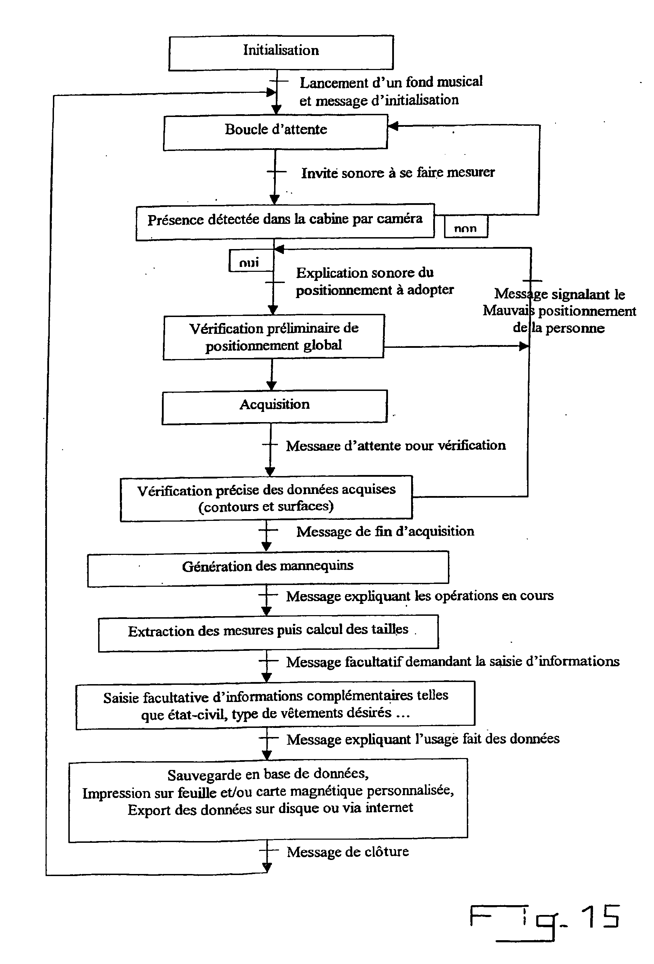

A titre d'exemple, un ordinogramme représentant le déroulement normal d'une séance d'acquisition est représenté sur la figure 15 annexée.

Bien entendu, l'invention n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention.

## Revendications

1. Installation automatique d'acquisition de formes tridimensionnelles et de prise de mensurations de sujets, notamment sous la forme d'une cabine destinée à des sujets humains, comprenant au moins une unité de projection de franges, en particulier de franges lumineuses, et au moins une unité de prise de vues rapide, ainsi qu'une unité de commande de l'installation et de traitement des vues acquises, intégrée ou séparée **caractérisée en ce que** lesdites unités (4, 5) de projection et de prise de vues sont regroupées dans une ou plusieurs tête(s) d'acquisition (3) et **en ce que** ladite installation (1) comprend également, d'une part, plusieurs marqueurs ou points de repérage fixes (7) et plusieurs surfaces de projection (8) de référence entourant la zone (9) d'acquisition et de mesure recevant le sujet (2) à saisir et situés dans le champ de vision de l'unité ou de chacune des unités (5) de prise de vues et, d'autre part, un moyen (10) de génération d'un mur de lumière temporaire à l'opposé de ladite au moins une tête d'acquisition (3) ou de chacune des têtes d'acquisitions (3) par rapport à la zone d'acquisition (9).

2. Installation selon la revendication 1, **caractérisée en ce que** la zone d'acquisition (9) consiste en un volume sensiblement parallélépipédique, les marqueurs (7) étant disposés à des emplacements connus de l'unité de commande et de traitement (6) selon plusieurs alignements latéraux dans la direction de prise de vue et dans deux plans parallèles espacés (P, P') délimitant les faces supérieure et inférieure dudit volume (9) et les surfaces de projection de référence (8), non déformantes et préférentiellement planes, consistant en des réglettes verticales reliant les deux plans (P, P') précités au niveau des quatre arêtes verticales de la zone d'acquisition (9), les informations fournies par les marqueurs (7) et les surfaces de projection (8) sur les différentes vues étant exploitées par l'unité (6) à chaque acquisition en vue de l'étalonnage de l'installation (1).

3. Installation selon l'une quelconque des revendications 1 et 2, **caractérisée en ce qu'**elle comporte deux têtes d'acquisition (3) situées en opposition de part et d'autre de la zone d'acquisition (9), **en ce que** chacune desdites têtes (3) englobe dans son champ de vision l'ensemble des marqueurs (7) et des surfaces de projection (8) et **en ce qu'**à chacune desdites têtes (3) est associé un moyen (10) de génération d'un mur de lumière correspondant, l'arrangement des marqueurs (7) et/ou des surfaces de projection (8) permettant de déterminer les extrémités supérieures et inférieures de la zone d'acquisition (9) et l'orientation de cette dernière.

4. Installation selon l'une quelconque des revendications 1 et 2, **caractérisée en ce qu'**elle comporte quatre têtes d'acquisition (3) regroupées en deux paires de têtes situées en opposition de part et d'autre de la zone d'acquisition (9), et **en ce qu'**une tête, située dans une position inférieure, de chacune des paires de têtes précitées englobe dans son champ de projection et de vision une partie inférieure du volume d'acquisition (9) et que l'autre tête, située sans une position supérieure, de chacune desdites paires de têtes englobe une partie supérieure dudit volume (9), complémentaire de et partiellement chevauchante avec la partie inférieure précitée, lesdites têtes inférieures, respectueusement supérieures, englobant, en outre, les marqueurs (7) inférieurs, respectivement supérieurs, et des portions inférieures, respectivement supérieures, des surfaces de projection (8) de référence.

5. Installation selon l'une quelconque des revendications 1 et 2, **caractérisée en ce qu'**elle comporte au moins trois têtes d'acquisition (3), ou au moins trois unités de prises de vues (5), disposées en Y dans la cabine formant l'installation (1).

6. Installation selon l'une quelconque des revendications 1 et 2, **caractérisée en ce qu'**elle comporte au moins quatre têtes d'acquisition (3), ou au moins quatre unités de prises de vues (5), disposées en X dans la cabine formant l'installation (1).

7. Installation selon l'une quelconque des revendications 1 à 6, **caractérisée en ce que** les moyens (10) de génération de mur de lumière consistent en des flashs électroniques dont le déclenchement est synchronisé avec celui de l'unité ou des unités de prise de vues (5) qui lui est (sont) opposée(s) par rapport à la zone d'acquisition (9), cette ou ces unité(s) (5) consistant chacune en une caméra vidéo numérique, par exemple du type CCD.

8. Installation selon l'une quelconque des revendications 1 à 7, **caractérisée en ce que** les franges alternées noires et blanches sont produites par les unités (4) au moyen de flashs électroniques associés à des objectifs de projection sans déformation géométrique, par exemple du type asphérique, et synchronisés en déclenchement avec les unités de prise de vues (5) des têtes d'acquisition (3) correspondantes, les franges noires présentant une largeur supérieure à celles des franges blanches ou lumineuses, le rapport des largeurs franges noires / franges blanches étant compris entre 1,1 et 1,5, préférentiellement d'environ 1,3.

9. Installation selon l'une quelconque des revendications 1 à 8, **caractérisée en ce que** chacun des réseaux de franges horizontales projetées comporte au moins deux franges blanches (FR1, FR2, FR3) mutuellement espacées, présentant des largeurs nettement supérieures à celles des autres franges, préférentiellement deux ou trois fois plus larges, et servant de franges de référence pour le repérage et l'identification des autres franges.

10. Installation selon la revendication 9, **caractérisée en ce que** chacun des réseaux de franges comporte trois franges de référence (FR1, FR2, FR3) disposées de manière asymétrique dans le motif du réseau concerné et/ou avec des espacements mutuels non identiques, une desdites franges de référence (FR1) étant localisée pour une projection à environ un tiers de la hauteur du volume formé par la zone d'acquisition (9) et au moins une autre (FR2) pour une projection à environ deux tiers de la hauteur du volume formé par ladite zone (9).

11. Procédé automatique d'acquisition quasi-instantanée de formes tridimensionnelles et de prise de mensurations de sujets, en particulier humains, notamment au moyen de l'installation selon l'une quelconque des revendications 1 à 4 et 7 à 10, **caractérisé en ce qu'**il consiste essentiellement, à placer ou faire placer un sujet (2) au niveau d'une zone d'acquisition (9), située dans une cabine ou analogue, ce sujet (2) étant placé dans une posture et/ou une position donnée et cette zone (9) comportant des marqueurs (7) et des surfaces de projection (8) de référence, situés dans les champs de vision de têtes d'acquisition (3) de vues dudit sujet (2) et constituant des repères d'étalonnage permanents, à réaliser l'acquisition d'une vue dite vue de face comprenant, d'une part, la prise d'une première image en vue de face du sujet (2) au moyen d'une première unité de prise de vues (5) en synchronisme avec une illumination créant un mur de lumière derrière le sujet (2) par l'intermédiaire d'un premier moyen (10) de génération de mur de lumière, pour l'acquisition d'un premier contour de silhouette en ombre chinoise et, d'autre part, la prise d'une seconde image de devant du sujet (2) au moyen de ladite première unité (5) de prise de vues en synchronisme avec la projection d'un réseau de franges horizontales au moyen d'une première unité de projection (4) sur la face avant du sujet (2), puis à réaliser l'acquisition d'une vue dite vue de derrière ou de dos prise dans une direction opposée à la direction de prise de vue de la vue de face, ladite acquisition comprenant, d'une part, la prise d'une première image en vue de dos du sujet (2) au moyen d'une seconde unité de prise de vues (5) en synchronisme avec une illumination créant un mur de lumière devant le sujet (2) par l'intermédiaire d'un second moyen (10) de génération de mur de lumière, pour l'acquisition d'un second contour de silhouette en ombre chinoise, et, d'autre part, la prise d'une seconde image de dos du sujet (2) au moyen de ladite seconde unité de prise de vues (5) en synchronisme avec la projection d'un réseau de franges horizontales au moyen d'une seconde unité de projection (4) sur la face arrière du sujet (2) et, enfin, à traiter, au moyen d'une unité (6) de commande et de traitement adapté, les images et les vues acquises par corrélation et exploitations des différentes informations fournies par elles en vue de la constitution d'une représentation tridimensionnelle du sujet (2), de l'extraction de mensurations et/ou de la classification du sujet (2) dans une ou des catégories prédéfinies.

12. Procédé selon la revendication 11, **caractérisé en ce que** les réseaux de franges projetés comprennent chacun des franges de référence (FR1, FR2, FR3) lumineuses réparties de manière asymétrique dans le motif du réseau concerné et présentant une largeur nettement supérieure à celles des autres franges et **en ce que** les prises d'images sont réalisées de manière séquentielle dans un intervalle de temps d'acquisition total suffisamment faible pour éviter toute prise en compte d'un mouvement éventuel du sujet (2).

13. Procédé selon l'une quelconque des revendications 11 et 12, **caractérisé en ce que** l'opération de traitement consiste notamment à détourer les images de surface acquises par projection de franges au moyen des images correspondantes acquises en ombre chinoise, puis à mettre en évidence et à localiser les franges sur les images par filtrage spatial, en déterminant, analysant et en localisant, éventuellement par extrapolation, les extrema supérieurs et inférieurs du signal recueilli le long d'une pluralité de lignes verticales, puis à identifier les différentes franges, le cas échéant après recomposition à partir de fragments de franges, et notamment les franges ou fragments de franges de référence (FR1, FR2, FR3), à numéroter ces franges ou fragments de référence et enfin à propager la numérotation à l'ensemble des franges contenu dans les représentations détourées du sujet (2).

14. Procédé selon la revendication 13, **caractérisé en ce que** l'identification des fragments de franges consiste à établir des relations d'adjacence verticale entre fragments consécutifs dans la direction verticale des vues et à qualifier ces relations, avec identification ou non de franges ou de fragments de franges réel(le)s, en fonction de la longueur ou du taux de recouvrement entre deux fragments adjacents, de la distance moyenne entre deux fragments adjacents et des couleurs respectives de ces derniers.

15. Procédé selon l'une quelconque des revendications 13 et 14, **caractérisé en ce que** la propagation de la numérotation des franges, après identification et numérotation des franges de référence (FR1, FR2, FR3), consiste à définir des chemins de recherche partant du haut de chaque représentation du sujet (2) sur les images de surfaces sous forme d'images à franges détourées et s'étendant vers le bas et/ou les côtés jusqu'aux extrémités des différentes ramifications de la représentation du sujet (2).

16. Procédé selon l'une quelconque des revendications 11 et 13, **caractérisé en ce que** la constitution de la représentation tridimensionnelle consiste, après identification et numérotation sur les images de surface détourées acquises par projection de franges en vue de face et en vue arrière, à générer des représentations, sous forme d'empilements de coupes, de différentes zones distinctes et complémentaires du sujet (2), zones déterminées au moyen de points caractéristiques localisés sur les contours obtenus à partir des vues en ombre chinoise, à assembler entre elles, pour chacune des zones distinctes précitées, les deux demi-coques correspondantes représentant en vue de devant et vue de derrière, sous forme d'empilements de coupes, une zone donnée du sujet (2) et, enfin, à reconstituer une représentation volumique tridimensionnelle totale par assemblage des empilements de coupes représentant les différentes zones distinctes du sujet (2).

17. Procédé selon l'une quelconque des revendications 11 à 16, **caractérisé en ce qu'**il consiste à réaliser, au cours de chaque acquisition de vue, une étape d'étalonnage automatique sur la base des informations fournies par les marqueurs (7) et les surfaces de projection (8) de référence sur les différentes images recueillies et exploitées par l'unité (6).

18. Procédé selon l'une quelconque des revendications 11 à 17, **caractérisé en ce qu'**il consiste également à réaliser, le cas échéant après rotation de 90° du sujet (2) autour d'un axe vertical, une prise de vue de profil par l'intermédiaire d'une unité de prise de vue (3) déclenché en synchronisme avec un moyen (10) de génération de mur de lumière opposé correspondant, afin d'acquérir le contour de la silhouette du sujet (2) en vue de profil.

## Claims

1. Automatic installation for the acquisition of three-dimensional shapes and for taking measurements of subjects, particularly in the form of a booth intended for human subjects, comprising at least one unit for projecting fringes, in particular light fringes, and at least one unit for rapidly taking views, as well as an integrated or separate control unit for the installation and processing of the acquired views, **characterised in that** said units (4, 5) for projection and taking views are combined into one or several acquisition head or heads (3) and **in that** said installation (1) also comprises, on the one hand, several markers or fixed reference points (7) and several reference projection surfaces (8) surrounding the acquisition and measuring zone (9) receiving the subject (2) to be acquired and located in the field of vision of the unit or each of the units (5) for taking views and, on the other hand, means (10) for generating a temporary light wall opposite said at least one acquisition head (3) or each of said acquisition heads (3) relative to the acquisition zone (9).

2. Installation according to claim 1, **characterised in that** the acquisition zone (9) has a substantially parallelepiped volume, the markers (7) being arranged at known locations of the control and processing unit (6) along several lateral alignments in the direction of taking the view and in two parallel spaced apart planes (P, P') which delimit the upper and lower surfaces of said volume (9) and the reference projection surfaces (8), which are non-deforming and preferably flat, consisting of vertical scales connecting the two aforementioned planes (P, P') at the level of four vertical edges of the acquisition zone (9), the information supplied by the markers (7) and the projection surfaces (8) on the different views being used by the unit (6) at each acquisition with the purpose of grading the installation (1).

3. Installation according to any one of claims 1 and 2, **characterised in that** it comprises two acquisition heads (3) located opposite one another on either side of the acquisition zone (9), **in that** each of the said heads (3) encloses in its field of vision all of the markers (7) and the projection surfaces (8) and **in that** each of said heads (3) is associated with a means (10) for generating a corresponding light wall, the arrangement of markers (7) and/or projection surfaces (8) allowing the determination of the upper and lower ends of the acquisition zone (9) and the orientation of the latter.

4. Installation according to any one of claims 1 and 2, **characterised in that** it comprises four acquisition heads (3) collected in two pairs of heads located opposite one another on either side of the acquisition zone (9), and **in that** one head, located in a lower position, of each of the pairs of aforementioned heads encloses in its field of projection and vision a lower portion of the acquisition volume (9) and the other head, located in an upper position, of each of said pairs of heads, encloses an upper portion of said volume (9), complementary to and partially overlapping the aforementioned lower portion, said lower heads, respectively upper heads, enclosing, in addition, lower markers (7) respectively upper markers, and lower portions, respectively upper portions, of the reference projection surfaces (8).

5. Installation according to any one of claims 1 and 2, **characterised in that** it comprises at least three acquisition heads (3), or at least three units (5) for taking views, arranged in a Y shape in the booth forming the installation (1).

6. Installation according to any one of claims 1 and 2, **characterised in that** it comprises at least four acquisition heads (3), or at least four units (5) for taking views, arranged in an X shape in the booth forming the installation (1).

7. Installation according to any one of claims 1 to 6, **characterised in that** the means (10) for generating a wall of light consists of electronic flashes the triggering of which is synchronised with that of the unit or units (5) for taking views which is opposite relative to the acquisition zone (9), said unit or units (5) each consisting of a digital video camera, for example of the CCD type.

8. Installation according to any one of claims 1 to 7, **characterised in that** the alternate black and white fringes are produced by the units (4) by means of electronic flashes associated with projection objectives without geometric deformation, for example of the aspherical type, and synchronised with respect to triggering with the units (5) for taking views of the corresponding acquisition heads (3), the black fringes having a width greater than that of the white fringes or luminous fringes, the ratio of the widths of the black fringes/white fringes being between 1.1 and 1.5, preferably about 1.3.

9. Installation according to any one of claims 1 to 8, **characterised in that** each of the networks of projected horizontal fringes comprises at least two white fringes (FR1, FR2, FR3) mutually spaced apart, having widths substantially greater than those of the other fringes, preferably two or three times greater, and serving as reference fringes for referencing and identifying the other fringes.

10. Installation according to claim 9, **characterised in that** each of the networks of fringes comprises three reference fringes (FR1, FR2, FR3) arranged asymmetrically in the design of the network in question and/or with non-identical mutual spacings, one of said reference fringes (FR1) being located for projection at about one third of the height of the volume formed by the acquisition zone (9) and at least one other fringe (FR2) for projection at about two-thirds of the height of the volume formed by said zone (9).

11. Automatic process for the virtually instantaneous acquisition of three-dimensional shapes and taking measurements of subjects, in particular humans, particularly by means of the installation according to any one of claims 1 to 4 and 7 to 10, **characterised in that** it consists essentially of placing or causing to be placed a subject (2) in an acquisition zone (9), located in a booth or the like, said subject (2) being put into a given pose and/or position and said zone (9) comprising markers (7) and reference projection surfaces (8) which are located in the fields of vision of the acquisition heads (3) of views of said subject (2) and constituting permanent standardisation references, for achieving the acquisition of a so-called front view comprising, on the one hand, taking a first front image of the subject (2) by means of a first unit (5) for taking views, synchronously with illumination creating a wall of light behind the subject (2) by means of a first means (10) for generating a wall of light, for obtaining a first silhouette contour, and, on the other hand, taking a second front image of the subject (2) by means of said first unit (5) for taking images synchronously with the projection of a network of horizontal fringes by means of a first projection unit (4) on the front side of the subject (2), then obtaining a so-called rear view in a direction opposite the direction of the front view, said acquisition comprising, on the one hand, taking a first rear view image of the subject (2) by means of a second unit (5) for taking views synchronously with illumination creating a wall of light in front of the subject (2) by means of a second means (10) for generating a wall of light, for the acquisition of a second silhouette contour, and, on the other hand, taking a second image of the back of the subject (2) by means of said second unit (5) for taking views synchronously with the projection of a network of horizontal fringes by means of a second projection unit (4) on the rear side of the subject (2) and, finally, processing, by means of a suitable control and processing unit (6), the images and views obtained by correlation and exploitation of the different information supplied thereby with a view to constructing a three-dimensional representation of the subject (2), for the extraction of measurements and/or for the classification of the subject (2) in one or more predetermined categories.

12. Process according to claim 11, **characterised in that** the projected networks of fringes each comprise luminous reference fringes (FR1, FR2, FR3) distributed asymmetrically in the design of the network in question and having a width substantially greater than that of the other fringes and **in that** the taking of images is performed in a sequential manner over a total interval of acquisition time that is sufficiently small to avoid the need to take into account any possible movement of the subject (2).

13. Process according to any one of claims 11 and 12, **characterised in that** the processing operation consists particularly in outlining surface images acquired by projection of fringes by means of corresponding images acquired in silhouette, then displaying and locating the fringes on the images by spatial filtration, by determining, analysing and locating, possibly by extrapolation, the upper and lower ends of the received signal along a plurality of vertical lines, then identifying the different fringes, accordingly after reconstruction from fragments of fringes, and particularly the reference fringes or fragments of fringes (FR1, FR2, FR3), counting said reference fringes or fragments of fringes and finally propagating the counting to all of the fringes contained in the outlined representations of the subject.

14. Process according to claim 13, **characterised in that** the identification of the fragments of fringes consists of establishing relationships of vertical adjacency between consecutive fragments in the vertical direction of the views and quantifying these relationships, with identification or non-identification of the fringes or fragments of real fringes, as a function of the length or size of overlap between two adjacent fragments, of the average distance between two adjacent fragments and the respective colours of the latter.

15. Process according to any one of claims 13 and 14, **characterised in that** the propagation of the counting of the fringes, after identification and counting the reference fringes (FR1, FR2, FR3), consists of defining search paths beginning at the top of each representation of the subject (2) on the surface images in the form of outlined fringe images and extending downwards and/or along the sides to the ends of the different ramifications of the representation of the subject (2).

16. Process according to any one of claims 11 and 13, **characterised in that** the construction of the three-dimensional representation consists, after identification and numbering on the outlined surface images acquired by projection of fringes in front and rear views, of generating representations, in the form of stacks of slices, of different separate and complementary zones of the subject (2), zones determined by means of characteristic points located on the contours obtained from silhouette views, assembling together, for each of the aforementioned separate zones, the two corresponding half shells representing the front and rear views, in the form of stacks of slices, the given zone of the subject (2) and, finally, reconstituting a complete three-dimensional volumetric representation by assembling the stacks of slices representing the different separate zones of the subject (2).

17. Process according to any one of claims 11 to 16, **characterised in that** it consists of carrying out, in the course of each acquisition of a view, a step of automatic standardisation on the basis of information supplied by the markers (7) and the reference projection surfaces (8) on the different images collected and used by the unit (6).

18. Process according to any one of claims 11 to 17, **characterised in that** it also consists of taking, according to the case, after a rotation of the subject (2) by 90° about a vertical axis, a profile view by means of a view-taking unit (3) triggered synchronously with a means (10) for generating a corresponding opposite wall of light, so as to acquire the contour of the silhouette of the subject (2) in profile view.

## Patentansprüche

1. Automatische Anlage zum Erfassen dreidimensionaler Formen und zur Aufnahme von Körpermaßen von Gegenständen, insbesondere in Form einer für menschliche Objekte bestimmten Kabine, welche mindestens eine Einheit zur Projektion von Streifen, insbesondere von Leuchtstreifen umfasst, und mindestens eine Einheit für Schnellaufnahmen sowie eine Einheit zum Steuern der Anlage und Bearbeiten der erfassten Aufnahmen, integriert oder getrennt umfasst, **dadurch gekennzeichnet, dass** die Einheiten (4, 5) zur Projektion und Aufnahme in einem oder mehreren Aufnahmeköpfen (3) zusammengefasst sind und dass die Anlage (1) ebenfalls einerseits mehrere Markierungen oder feste Markierungspunkte (7) und mehrere Referenzprojektionsflächen (8), welche den Aufnahme- und Messbereich (9) umgeben, der den zu erfassenden Gegenstand (2) aufnimmt und im Blickfeld der Einheit oder von jeder der Aufnahmeeinheiten (5) gelegen ist, und andererseits ein Mittel (10) zum Erzeugen einer temporären Lichtmauer gegenüber von mindestens einem Aufnahmekopf (3) oder jedem der Aufnahmeköpfe (3) in Bezug zum Aufnahmebereich (9) umfasst.

2. Anlage gemäß Anspruch 1, **dadurch gekennzeichnet, dass** der Aufnahmebereich (9) aus einem etwa quaderförmigen Raum besteht, wobei die Markierungen (7) an bekannten Stellen der Steuer- und Bearbeitungseinheit (6) entlang mehrerer seitlicher Fluchten in der Aufnahmerichtung und in zwei parallelen, beabstandeten Ebenen (P, P'), welche die obere und untere Fläche des Raumes (9) und die Referenzprojektionsflächen (8) nicht verformend und vorzugsweise eben begrenzen, angeordnet sind, bestehend aus vertikalen, die beiden vorher erwähnten Ebenen (P, P') auf dem Niveau der vier vertikalen Kanten des Aufnahmebereichs (9) verbindenden Regletten, die durch die Markierungen (7) und die Projektionsflächen (8) gelieferten Informationen über die verschiedenen Ansichten werden durch die Einheit (6) an jede Aufnahme im Hinblick auf die Kalibrierung der Anlage (1) ausgewertet.

3. Anlage gemäß einem der Ansprüche 1 und 2, **dadurch gekennzeichnet, dass** sie zwei, hinsichtlich dem Aufnahmebereich (9) einander gegenüberliegend angeordnete Aufnahmeköpfe (3) umfasst, dass jeder der Köpfe (3) die Gesamtheit der Markierungen (7) und die Projektionsflächen (8) in seinem Blickfeld umfasst und dass jeder der Köpfe (3) mit einem entsprechenden Mittel (10) zur Erzeugung einer Lichtmauer verbunden ist, wobei die Anordnung der Markierungen (7) und/oder der Projektionsflächen (8) das Bestimmen der oberen und unteren Enden des Aufnahmebereichs (9) und dessen Orientierung ermöglicht.

4. Anlage gemäß einem der Ansprüche 1 und 2, **dadurch gekennzeichnet, dass** sie vier, in zwei Paaren von Köpfen unterteilte und hinsichtlich dem Aufnahmebereich (9) einander gegenüber angeordnete Aufnahmeköpfe (3) umfasst, und dass ein in einer unteren Position angeordneter Kopf von jedem der erwähnten Kopfpaare in seinem Projektions- und Blickfeld einen unteren Teil des Aufnahmeraums (9) umfasst und dass der andere, in einer oberen Position angeordnete Kopf von jedem der Kopfpaare einen oberen, zusätzlich und teilweise mit dem erwähnten unteren Teil überlappenden Teil des Raums (9) umfasst, wobei die unteren beziehungsweise oberen Köpfe außerdem die unteren, beziehungsweise die oberen Markierungen (7) und die unteren beziehungsweise oberen Anteile der Referenzprojektionsflächen (8) umfassen.

5. Anlage gemäß einem der Ansprüche 1 und 2, **dadurch gekennzeichnet, dass** sie mindestens drei Aufnahmeköpfe (3) oder mindestens drei in Form eines Y in der die Anlage (1) bildenden Kabine angeordneten Aufnahmeeinheiten (5) umfasst.

6. Anlage gemäß einem der Ansprüche 1 und 2, **dadurch gekennzeichnet, dass** sie mindestens vier Aufnahmeköpfe (3) oder mindestens vier in Form eines X in der die Anlage (1) bildenden Kabine angeordneten Aufnahmeeinheiten (5) umfasst.

7. Anlage gemäß einem der Ansprüche 1 bis 6, **dadurch gekennzeichnet, dass** die Mittel (10) zur Erzeugung der Lichtmauer aus Elektronenblitzgeräten bestehen, deren Auslöser mit demjenigen (denjenigen) Aufnahmeeinheit(en) (5), der (die) ihm hinsichtlich des Empfangsbereichs (9) gegenüber angeordnet ist (sind), synchronisiert ist (sind), wobei diese Einheit(en) (5) jeweils aus einer Digitalkamera, zum Beispiel vom Typ CCD bestehen.

8. Anlage gemäß einem der Ansprüche 1 bis 7, **dadurch gekennzeichnet, dass** die schwarz und weiß alternierenden Streifen durch Einheiten (4) mittels der mit den Projektionsobjektiven verbundenen Elektronenblitzgeräten ohne geometrische beispielsweise torische Verformungen, erzeugt werden, und der Auslöser mit den Aufnahmeeinheiten (5) der entsprechenden Aufnahmeköpfe (3) synchronisiert ist, wobei die schwarzen Streifen eine größere Breite aufweisen als die weißen oder leuchtenden Streifen, wobei das Breitenverhältnis schwarze/weiße Streifen zwischen 1,1 und 1,5, vorzugsweise ungefähr 1,3 beträgt.

9. Anlage gemäß einem der Ansprüche 1 bis 8, **dadurch gekennzeichnet, dass** jedes der Raster aus projizierten horizontalen Streifen mindestens zwei mit Abstand angeordnete weiße Streifen (FR1, FR2, FR3) umfasst, welche deutlich größere, vorzugsweise zwei oder drei Mal so große Breiten wie die anderen Streifen aufweisen und den Bezugsstreifen zum Markieren und Identifizieren der anderen Streifen dienen.

10. Anlage gemäß Anspruch 9, **dadurch gekennzeichnet, dass** jedes der Streifenraster drei im betreffenden Rastermuster asymmetrisch und/oder mit zueinander nicht identischen Abständen angeordneten Bezugsstreifen (FR1, FR2, FR3) umfasst, wobei einer der Bezugsstreifen für eine Projektion von ungefähr einem Drittel der Höhe des durch den Aufnahmebereich (9) gebildeten Raums und mindestens ein anderer (FR2) für eine Projektion von ungefähr zwei Dritteln der Höhe des durch den Bereich (9) gebildeten Raum angeordnet ist.

11. Automatisches Quasi-Schnappschuss-Verfahren zum Erfassen dreidimensionaler Formen und zur Aufnahme der Körpermaße von Gegenständen, insbesondere menschlicher Körper, vor allem mittels einer Anlage gemäß einem der Ansprüche 1 bis 4 und 7 bis 10, **dadurch gekennzeichnet, dass** es hauptsächlich besteht aus dem Platzieren oder Platzieren lassen eines Gegenstandes (2) auf dem Niveau eines in einer Kabine oder ähnlichem gelegenen Aufnahmebereichs (9), wobei dieser Gegenstand (2) in einer vorgegebenen Stellung und/oder Position platziert ist und dieser Bereich (9) Markierungen (7) und Referenzprojektionsflächen (8) umfasst, welche in den Blickfeldern der Aufnahmeköpfe (3) des Gegenstandes (2) angeordnet sind und permanente Kalibrierungsmarkierungen bilden, aus dem Realisieren einer Aufnahme einer Vorderansicht, welche einerseits die Aufnahme eines ersten Bildes der Vorderansicht des Gegenstandes (2) mittels einer ersten Aufnahmeeinheit (5) synchron mit einer Beleuchtung, die durch ein erstes Mittel (10) zur Erzeugung einer Lichtmauer eine Lichtmauer hinter dem Gegenstand (2) zum Erfassen einer ersten Umrisskontur als Schattenbild erzeugt, und andererseits die Aufnahme eines zweiten Bildes der Vorderseite des Gegenstandes (2) mittels der ersten Aufnahmeeinheit (5) synchron mit der Projektion eines horizontalen Streifenrasters mittels einer ersten Projektionseinheit (4) auf die vordere Fläche des Gegenstandes (2) umfasst, dann das Realisieren der Aufnahme der Hinter- oder Rückansicht, aufgenommen in einer der Aufnahmerichtung der Vorderaufnahme entgegengesetzten Richtung, wobei die Aufnahme einerseits die Aufnahme eines ersten Bildes der Rückansicht des Gegenstandes mittels einer zweiten Aufnahmeeinheit (5) synchron mit einer Beleuchtung, die durch ein zweites Mittel (10) zur Erzeugung einer Lichtmauer eine Lichtmauer vor dem Gegenstand (2) zum Erfassen einer zweiten Umrisskontur als Schattenbild erzeugt, und andererseits die Aufnahme eines zweiten Bildes der Rückansicht des Gegenstandes (2) mittels der zweiten Aufnahmeeinheit (5) synchron mit der Projektion eines horizontalen Streifenrasters mittels einer zweiten Projektionseinheit (4) auf die hintere Fläche des Gegenstandes (2), und schließlich aus dem Bearbeiten der Bilder und Aufnahmen durch Korrelieren und Auswerten der verschiedenen durch diese gelieferten Informationen im Hinblick auf die Zusammensetzung einer dreidimensionalen Darstellung, dem Extrahieren von Körpermaßen und/oder dem Klassifizieren des Gegenstandes (2) in (eine) vordefinierte Kategorie(n) mittels einer angepassten Steuerungs- und Bearbeitungseinheit (6).

12. Verfahren gemäß Anspruch 11, **dadurch gekennzeichnet, dass** jedes der projizierten Streifenraster leuchtende Referenzstreifen (FR1, FR2, FR3) umfasst, welche asymmetrisch in dem betreffenden Rastermuster verteilt sind und eine deutlich höhere Breite als die anderen Streifen aufweisen, und dass die Aufnahmen sequentiell in einem ausreichend kurzen Gesamtaufnahmezeitraum durchgeführt werden, um jede Berücksichtigung einer eventuellen Bewegung des Gegenstandes (2) zu vermeiden.

13. Verfahren gemäß einem der Ansprüche 11 und 12, **dadurch gekennzeichnet, dass** der Bearbeitungsvorgang vor allem besteht aus dem Schneiden der Flächenbilder, welche durch Projektion der Streifen mittels der entsprechenden, als Schattenbilder erfassten Bilder aufgenommen wurden, dann dem Hervorheben und Lokalisieren der Streifen auf den Bildern durch räumliches Filtern, wobei die oberen und unteren Extrema des empfangenen Signals gegebenenfalls durch Extrapolation entlang einer Vielzahl von vertikalen Linien analysiert und lokalisiert werden, dann dem Identifizieren der verschiedenen Streifen und insbesondere der Referenzstreifen oder -streifenfragmente (FR1, FR2, FR3), gegebenenfalls nach Neuzusammensetzung ab den Streifenfragmenten, dem Nummerieren dieser Streifen oder Referenzfragmente und schließlich dem Ausweiten der Nummerierung auf die Gesamtheit der in den Ausschnitten der Darstellungen des Gegenstandes (2) enthaltenen Streifen.

14. Verfahren gemäß Anspruch 13, **dadurch gekennzeichnet, dass** das Identifizieren der Streifenfragmente besteht aus dem Aufbauen der vertikal angrenzenden Beziehungen zwischen den aufeinander folgenden Fragmenten in vertikaler Richtung der Aufnahmen und dem Qualifizieren dieser Beziehungen mit dem Identifizieren oder Nicht-Identifizieren der Ist-Streifen oder -Streifenfragmente in Abhängigkeit von der Länge oder den Abdeckungsgraden zwischen zwei angrenzenden Fragmenten, dem mittleren Abstand zwischen zwei angrenzenden Fragmenten und ihrer jeweiligen Farben.

15. Verfahren gemäß einem der Ansprüche 13 und 14, **dadurch gekennzeichnet, dass** das Ausweiten der Streifennummerierung nach dem Identifizieren und Nummerieren der Referenzstreifen (FR1, FR2, FR3) besteht aus dem Definieren der Suchwege, ausgehend vom oberen Teil jeder Darstellung des Gegenstandes (2) auf den Flächenbildern in Form von Streifenbildausschnitten und nach unten und/oder seitlich bis zu den Armen der verschiedenen Verzweigungen der Darstellung des Gegenstandes (2) angeordnet.

16. Verfahren gemäß einem der Ansprüche 11 und 13, **dadurch gekennzeichnet, dass** die Zusammensetzung der dreidimensionalen Darstellung nach dem Identifizieren und Nummerieren auf den durch Streifenprojektion der Vorder- und Hinteransicht erfassten Flächenbildausschnitten besteht aus dem Erzeugen der Darstellungen in Form von Schnittstapeln verschiedener getrennter und ergänzender Bereiche des Gegenstands (2), mittels charakteristischer, auf den ausgehend von den Schattenbildaufnahmen erhaltenen Konturen lokalisierten Punkten bestimmter Bereiche, dem Zusammensetzen untereinander für jede der erwähnten, unterschiedlichen Bereiche, die beiden entsprechenden Halbkörper weisen eine Vorder- und Hinteransicht in Form von Schnittstapeln, einen angegebenen Bereich des Gegenstands (2) auf und, schließlich, dem Rekonstruieren einer räumlichen dreidimensionalen Gesamtdarstellung durch Zusammensetzen der Stapelschnitte, welche die verschiedenen unterschiedlichen Bereiche des Gegenstandes (2) darstellen.

17. Verfahren gemäß einem der Ansprüche 11 bis 16, **dadurch gekennzeichnet, dass** es im Verlauf jeder Aufnahme aus dem Durchführen eines automatischen Kalibrierungsschritt auf der Basis der durch die Markierungen (7) und die Referenzprojektionsflächen (8) auf den verschiedenen, durch die Einheit (6) aufgenommenen und entwickelten Bilder gelieferten Informationen besteht.

18. Verfahren gemäß einem der Ansprüche 11 bis 17, **dadurch gekennzeichnet, dass** es ebenfalls aus dem Realisieren einer Seitenaufnahme, gegebenenfalls nach einer 90°-Drehung des Gegenstandes (2) um eine vertikale Achse, mittels einer Aufnahmeeinheit (3) synchron ausgelöst mit einem Mittel (10) zur Erzeugung einer entsprechend entgegengesetzten Lichtmauer, um die Umrisskontur des Gegenstandes (2) in der Seitenansicht zu erhalten.
